Europäisches Patentamt

European Patent Office

Office européen des brevets

(19)

(11) Veröffentlichungsnummer: **0 000 504**
**B1**

(12) **EUROPÄISCHE PATENTSCHRIFT**

(45) Veröffentlichungstag der Patentschrift: **19.05.82**

(51) Int. Cl.³: **A 61 B 5/04**

(21) Anmeldenummer: **78100372.8**

(22) Anmeldetag: **12.07.78**

(54) Schaltungsanordnung zur Detektion und Registrierung der Uterusaktivität.

(30) Priorität: **13.07.77 DE 2732160**

(43) Veröffentlichungstag der Anmeldung:
**07.02.79 Patentblatt 79/3**

(45) Bekanntmachung des Hinweises auf die Patenterteilung:
**19.05.82 Patentblatt 82/20**

(84) Benannte Vertragsstaaten:
**CH DE FR GB NL SE**

(56) Entgegenhaltungen:
**GYNÉOLOGIE ET OBSTÉTRIQUE Band 55, Nr. 2, 1956, Seiten 153—175, C. Sureau: "Étude de l'activité électrique de l'utérus au cours du travail"**
**IEEE TRANSACTIONS ON BIOMEDICAL ENGINEERING, Band BME—15, Nr. 1, Januar 1968, New York (USA) J. H. VAN BEMMEL "Detection of weak foctal electrocardiograms by autocorrelation and crosscorrelation of envelopes", Seiten 17—23**
**MEDICAL AND BIOLOGICAL ENGINEERING, Band 8, Nr. 3, Mai 1970, London (GB) S.**

(73) Patentinhaber: **BIOTRONIK Mess- und Therapiegeräte GmbH & Co Ingenieurbüro Berlin Sieversufer 8 D-1000 Berlin 47 (DE)**

(72) Erfinder: **Nagel, Joachim Schobertweg 3A D-8520 Erlangen (DE)**

(74) Vertreter: **Christiansen, Henning, Dipl.-Ing. Unter den Eichen 108a D-1000 Berlin 45 (DE)**

(56) Entgegenhaltungen:
**JOHANSSON, L. E. LARSSON und R. OERTENGREN "An automated method for the frequency analysis of myoelectric signals evaluated by an investigation of the spectral changes following strong sustained contractions", Seiten 257—264**
**MEDICAL AND BIOLOGICAL ENGINEERING, Band Nr. 10, Nr. 4, Juli 1972, London (GB) H. GARLAND, R. W. ANGEL und R. D. MELEN "A state variable averaging filter for electromyogram processing", Seiten 559—560**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

OBSTETRICS AND GYNECOLOGY, Band 37, Nr. 2, Februar 1971, Hagerstown (USA) G. WOLFS, M. VAN LEEUWEN, H. ROTTINGHUIS und J. Th. F. BOELES "An electromyographic study of the human uterus during labor", Seiten 241—246

## Schaltungsanordnüng zur Detektion und Registrierung der Uterüsaktivität

Die Erfindung betrifft eine Schaltungsanordnüng zur Detektion und Registrierung der Uterusaktivität mittels elektrischer Signale wie sie im Oberbegriff des Patentanspruchs 1 angegeben ist.

Die Überwachung und Auswertung der Uterusaktivität, welche eine Beurteilung der Wehentätigkeit ermöglicht, ist in der perinatalen Medizin wichtig zur Beobachtung des Verlaufs der Schwangerschaft und zur Beurteilung des Zustands des Feten.

Zur Ermittlung und Aufzeichnung des intrauterinen Drucks als Maß für die Uterusaktivität in Form des Tokogramms sind bisher im wesentlichen zwei Verfahren, nämlich ein internes und eine externes Verfahren, benutzt worden. Beiden Verfahren ist gemeinsam, daß der uterine Druck mittels Druckaufnehmern am Körper gemessen wird.

Bei der internen Tokographie wird zur Druckbestimmung eine mit Flüssigkeit gefüllter sogenannter "Open-End-Katheter" in die Amnionhöhle oder zwischen Fruchtblase und Uteruswand eingeführt. Nachdem eine Nullabgleich durchgeführt wurde, läßt sich mit diesem Verfahren der absolute Druck des Uterus bestimmen.

Das Verfahren der internen Tokographie wird wegen der schwierigen und nicht risikofreien Handhabung vor vielen Ärzten abgelehnt. Darüber hinaus beweisch die jüngsten Untersuchungen eine Beeinflussung des Geburtsvorgangs durch die Applikation des Katheters, weswegen dieses Verfahren gerade bei drohenden Frühgeburten, bei denen eine Überwachung der Wehentätigkeit von besonderer Bedeutung ist, nicht angewandt werden sollte.

Die externe Tokographie beruht auf einem von Rech schon im Jahre 1934 angegebenen Verfahren. Ein Druckaufnehmer, der normalerweise als Dehnungsmeßstreifen ausgebildet ist, wird mit einem elastischen Gurt auf dem Abdomen der Schwangeren befestigt. Durch die infolge Uteruskontraktionen verursachten Hubänderungen eines Taststiftes, der mechanisch auf den Dehnungsmeßstreifen wirkt, wird ein elektrisches Signal gewonnen, das ein Maß für die Wehentätigkeit darstellt und in seinem zeitlichen Verlauf von dem behandelnden Arzt ausgewertet wird.

Das Verfahren der externen Tokographie weist den Nachteil auf, daß die indirekte Methode der Druckaufnahme zahlreichen Störeinflüssen ausgesetzt ist, welche das Meßergebnis verfälschen. Derartige Störeinflüsse stellen zum Beispiel schon die mit der Atmung einhergehenden Hubänderungen dar. Dem noninvasiven Verfahren liegt die Annahme zugrunde, daß der am Abdomen mittels des Taststiftes festgestellte und registrierte Druck eine hinreichende Näherung des intrauterinen Druckes darstellt. Mit dieser Messung läßt sich jedoch keine ausreichend genaue Information über den Verlauf der Uterusaktivität gewinnen.

Beide Verfahren weisen damit bei der routinemäßigen klinischen Anwendung erhebliche Nachteile auf, so daß sie nur selten angewendet werden.

Es sind aber auch bereits Versuche unternommen, worden, aus elektrischen Signalen Rückschlüsse auf die Uterusaktivität zu ziehen, So ist aus GYNÉCOLOGIE ET OBSTÉTRIQUE, Band 55, Nr. 2, 1956, Seiten 153 bis 175, C. SUREAU, "Étude de l'activité électrique de l'utérus au cours du travail", bekannt, die elektrische Aktivität des Uterus mittels im abdominalen Bereich extern abgeleiteter Signale zu erfassen (aaO., Seite 156, Abschnitt "Électrodes"). An anderer Stelle wird auch erwähnt, daß bereits durch RC-gekoppelte Verstärker (AC-System) "sehr schnelle" Aktivitäten erfaßt und graphisch dargestellt wurden. Der betroffene Frequenzbereich lag dabei aber in Wirklichkeit sehr niedrig, d.h. zwischen 1/3 und 2 Hz (aaO., Seite 154, Absätze 3 und 4) und die Auswertung der Messungen bei diesen Verfahren ließ eine umkehrbar eindeutige Zuordnung von Meßergebnissen und Uterusaktivität nicht zu.

Aus OBSTETRICS AND GYNECOLOGY, Band 37, Nr. 2, Februar 1971, G. WOLFS, M. VAN LEEUWEN, H. ROTTINGHUIS und J. Th. F. BOELES, "An electromyographic study of the human uterus during labor", Seiten 241 bis 246, ist ebenfalls die elektromyographische Erfassung der elektrischen Aktivität des Uterus bekannt. Diese wird allerdings über interne bipolare Elektroden vorgenommen. Auch bei dieser Arbeit wurde festgestellt, daß eine klare Beziehung zwischen der aus häufigen Entladungsfolgen bestehenden elektrischen Aktivität des Uterus und dem Verlauf des intrauterinen Drucks nicht anzunehmen ist (Seite 245, Abschnitt "Discussion"). Das vorgenannte Verfahren hat neben seinen ungesicherten Ergebnissen den Nachteil, daß eine Vielzahl von zu intrauterinen Elektroden führenden elektromyographischen Zuleitungen vorgesehen sein muß, um die einzelnen Aktivitätszentren zu erfassen und mittels einer Aussage über die Reizausbreitung Klarheit über die Aktivität des gesamten Uterus zu gewinnen.

Der in Anspruch 1 angegebenen Erfindung liegt dem gegenüber die Aufgabe zugrunde, eine die Patientin wenig belästigende Vorrichtung der eingangs genannten Art anzugeben, welche dem Arzt in reproduzierbarer und in für ihn leicht interpretierbarer Weise ein zuverlässiges Ergebnis liefert.

Diese Aufgabe wird durch die Maßnahmen gemäß Anspruch 1 gelöst.

Die Erfindung geht davon aus, daß auch die intrauterinen Druckänderungen direkt durch Muskelkontraktionen hervorgerufen werden, und die mit diesen Kontraktionen verbundenen elektrischen Felder elektromyographisch an der Hautoberfläche meßbar sind. Ein Maß für die Intensität der Kontraktionen ist hier nicht direkt der gemessene Spannungsverlauf, sondern, wie mit der Erfindung gefunden wurde, ein Mittelwert der impulsförmigen Wechselspannungsanteile der gemessenen Signale in einem ganz bestimmten Frequenzintervall, das gegenüber dem bisher zur elektrischen Messung der Uterusaktivität verwendeten Frequenzintervall nach oben verschoben ist. Unter Intensität wird dabei ein Wert verstanden, der für den Betrag der Amplitude des Wechselspannungsanteils repräsentativ ist und beispielsweise durch Gleichrichtung des Wechselspannungsanteils bzw. Quadrierung im Sinne einer Leistungsermittlung gewonnen werden kann. Das augegebene Signal verläuft damit im wesentlichen mindestens ähnlich wie eine Einhüllende des elektro-myographisch erfaßten Signals.

Es ist damit erstmals eine Möglichkeit gegeben, den relativen uterinen Druck als Maß für die Uterusaktivität rein äußerlich zuverlässig, mit einem Verfahren zu messen, das das Wohlbefinden der Patientin nicht beeinträchtigt. Da das mittels Elektroden abgenommene Signal elektrischer Natur ist, ist eine Verstärkung und Weiterverarbeitung mit einer anschließenden graphischen Darstellung des zeitlichen Signalverlaufs ohne Schwierigkeiten möglich. Insbesondere ein Quadrieren läßt sich mit schaltungstechnischen Mitteln—vorzugsweise wenn eine näherungsweise Ermittlung ausreichend ist—in einfacher Weise ausführen.

Es ist zwar aus der Entgegenhaltung "MEDICAL AND BIOLOGICAL ENGINEERING", Band 10, Nr. 4, Juli 1972, Seite 559, zur Anwendung für elektromyographische Signale, die Informationen über das physiologische Verhalten der unter der Hautoberfläche liegenden Muskulatur geben sollen, eine Anordnung bekannt, die zum Teil mit derjenigen übereinstimmt, wie sie erfindungsgemäß zur Ermittlung der Uterusaktivität herangezogen werden soll. Die dabei benutzte bekannte Bezeihung zwischen der Muskelspannung und dem Elektromyogramm, die von Lippold im Jahre 1952 aufgestellt wurde, bezeiht sich aber ausschließlich auf quergestreifte Muskulatur, wie sie zum Antrieb von Gliedmaßen unter der Hautoberfläche angeordnet ist. Die angegebene lineare Beziehung ist dabei lediglich für den Fall der sogenannten isometrischen kontraktion zutreffend.

Demgegenüber handelt es sich bei der Wehenmessung um die Auswertung der Aktionspotentiale glatter Muskulatur, die von gänzlich anderer Art sind. Quergestreifte und glatte Muskulatur unterscheiden sich dabei nicht nur in ihrem Aufbau, sondern auch in ihrem Reizausbreitungs- und Signalverhalten. Während quergestreifte Muskulatur durch nervale Impulse insgesamt erregt wird, weist die Uterusmuskulatur autonome Erregungsbildung und myogene Erregungsleitung auf, die nicht den gesamten Muskel gleichzeitig aktiviert. Unterschiedliche Muskelbereiche können dabei verschiedene Erregungszustände aufweisen.

Ein weiterer fundamentaler Unterschied besteht darin, daß bei der vorgenannten bekannten Bezeihung eine mechanische Muskelspannung zugrundegelegt wird, die räumlich gleichgerichtet verläuft. Bei der Erfindung bildet das ausgegebene Signal jedoch kein Maß für eine aufgrund eindimensional gerichteter Kräfte ermittelte Größe, sondern stellt mit dem intrauterinen Druck eine Bezeihung zu einer Erscheinung her, der ein dreidimensionaler mechanischer Spannungszustand mit in allen Raumrichtungen wirkenden Muskelkräften zugrundeliegt.

Wegen der andersartigen Voraussetzungen und der mit den bisherigen Apparaturen erhaltenen, grundsätzlich verschiedenen Signale, die ihre Amplitudenmaxima in einem sehr niedrigen Frequenzbereich (unterhalb von 2 Hz) haben, wurden Versuche zur externen Erfassung der Uterusaktivität mit Anordnungen, wie sie zur elektromyographischen Erfassung der von quergestreifter Muskulatur ausgehenden Signale benutzt wurden, vor der Erfindung für aussichtslos gehalten. Mit der Erfindung ist vielmehr eine Korrektur des bis dahin gültigen Modells der elektrischen Uterusaktivität verbunden.

Besonders vorteilhaft ist bei einem auf der erfindungsgemäßen Vorrichtung basierenden medizinischen Untersuchungsgerät, daß es sich wegen seiner einfachen Bedienbarkeit und sicheren Erfassung der zu emittelnden Signale insbesondere für Routineuntersuchungen eignet, so daß sich nicht nur im klinischen Bereich ein weites Anwendungsfeld ergibt. Da das Meßergebnis mit dem der herkömmlichen mechanisch arbeitenden Tokographen vergleichbar ist, ergeben sich bezüglich der Interpretation der Ergebnisse für den behandelnden Arzt keinerlei Schwierigkeiten. Vorteilhaft ist weiterhin, daß in dem abdominal abgeleiteten Elektromyogramm die maternellen und fetalen Herzsignale zwar als Störsignale enthalten sind, diese aber gegebenenfalls ausgefiltert und getrennt ausgewertet werden können.

Außerdem ist bei der erfiedungsgemäßen Vorrichtung günstig, daß die in der Messung enthaltenen und so dem Arzt zugänglichen Informationen umfangreicher sind als es bei der Verwendung von Druckaufnehmern der Fall ist. Da die Befestigung von Hautelektroden wesentlich einfacher vorzunehmen ist als die Anbringung von mechanischen Druckaufnehmern, wird die Ermittlung der Uterustätigkeit für den Arzt wesentlich erleichtert. Mit der Verringerung der pro Patientin aufzuwendenden Untersuchungszeit ist auch eine Senkung der Behandlungskosten verbunden.

Bei der erfindungsgemäßen Vorrichtung ist es möglich, durch entsprechende Plazierung der Elektroden die Aktivität des gesamten Uterus sowie der Bauchmuskulatur zu erfassen. Aus der Form der emittelten und dargestellten Wehenkurven lassen sich Rückschlüsse auf die Erregung und Ausbreitung der Kontraktionen ziehen. Auf diese Weise kann der Arzt auch frühzeitig Motilitätsstörungen, wie beispielsweise Inkoordination, erkennen.

Die Auswertung der Myosignale kann in vorteilhafter Weise durch Verwendung eines entsprechenden Bandfilters in einem Frequenzbereich von ca. 150 bis 250 Hz vorgenommen werden. Dabei ist es günstig, daß die bei der Erfassung der Uterusmotilität störenden Signalanteile von fetalem und maternellem Elekrokardiogramm in diesem Frequenzbereich amplitudemäßig kleiner sind als die auszuwertenden Myopotentiale und daher das Ergebnis nur wenig beeinflussen.

Eine weitere Möglichkeit bei der Ermittlung der Uterustätigkeit störende Signale in ihrer Auswirkung zu vermindern besteht darin, die bei der Mittelwertbildung (durch Tiefpaßfilterung) des gleicherichteten oder quadrierten Signals benutzte Zeitkonstante hinreichend groß zu wählen. In diesem Fall kann (bei einer Zeitkonstante von mehreren Sekunden) sogar derjenige Frequenzbereich des myographisch aufgenommenen Signals von ungefähr 15 bis 40 Hz herangezogen werden, in dem die Herzsignale ihr Maximum haben. Die Auswahl des auszuwertenden engeren Frequenzbereiches und dessen Breite innerhalb eines Gesamtbereiches von ungefähr 10 bis 300 Hz hängt davon ab, ob die statischen oder dynamischen Anteile der Muskelkontraktionen bevorzugt ausgegeben werden sollen und in welchem Umfang der Einfluß der Bauchmuskulatur (Anspannung bei Preßwehen) im Ergebnis erscheinen soll.

Wird bei der Signalaufnahme durch entsprechende Filtermittel der Frequenzbereich auf denjenigen der Herzsignale begrenzt, so können diese Signale sowohl zur Emittlung der Wehentätigkiet als auch zur Auswertung der Herzsignale verwendent werden.

Die Ausgabe der gemittelten Signale, die ein Maß für den intrauterinen Druck bilden, kann in vorteilhafter Weise, beisielsweise über einen angeschlossenen Schreiber, in logarithmischer Darstellung erfolgen, so daß ein großer Amplitudenbereich erfaßt wird. Der Verstärkungsfaktor und die Nullinie müssen dabei nicht nachjustiert werden, so daß eine einfache Bedienbarkeit gewährleistet ist.

Wird das gemittelte Signal potenziert, so werden die Maxima der Wehenkurven besonders deutlich sichtbar.

Die Signalverarbeitung kann in besonders einfacher Weise mittels analog abeitender Baugruppen wie Operationsverstärkern erfolgen. Wird die erfindungsgemäße Vorrichtung mit einer solchen zur Ermittlung der Herztätigkeit zusammengefaßt, so wird die Signalverarbeitung günsigerweise digital, vorzugsweise mittels Mikroprozessoren, vorgenommen.

Ihre besonderen Vorzüge entfaltet die erfindungsgemäße Vorrichtung in dem Fall, daß mittels abdominal angebrachter Elektroden gleichzeitig sowohl die Uterus- als auch die fetale (bzw. die maternelle) Herztätigkeit betreffende Signale abgeleitet werden bzw. die aufgenommenen Signale in einem gemeinsamen Gerät ausgewertet werden. Damit besteht die Möglichkeit, im Rahmen perinataler Untersuchungen mit einer einzigen, die Patientin wenig belastenden Messung umfangreiche Daten zu gewinnen, welche den Arzt in die Lage versetzen, schnell ein genaues Bild über den Zustand des Feten und der Patientin zu gewinnen, so daß er die erforderlichen Maßnahmen unverzüglicheinleiten kann.

Wird dabei der auszuwertende Frequenzbereich, wie oben dargestellt, unter Wahl einer entsprechend großen Zeitkonstanten für die Mittelung so gelegt, daß er den Bereich der von den Herzsignalen eingenommen Frequenzen (mit-) umfaßt, so sind keine weiteren Filtermittel erforderlich, um die Signale für die Ermittlung der Uterustätigkeit von denen für die Ermittlung der Herztätigkeit zu trennen.

Wenn Mittel vorgesehen sind, um die Signalauswertung beeinträchtigende wiederkehrende Störsignale nach einem ersten Kriterium zu erkennen, in ihrem Amplitudenverlauf in einem Speicher festzuhalten und, wenn das Auftreten eines Störsignals im Eingangssignal entdeckt ist, von diesem amplitudenmäßig phasenrichtig zu subtrahieren, so ist damit die Möglichkeit gegeben, die Auswertung beeinträchtigende Signalanteile, wie den maternellen QRS-Komplex, sowohl hinsichtlich der die Uterus-, als auch hinsichtlich der die Herztätigkeit betreffenden Signale durch gemeinsame Mittel zu beseitigen. Die weitere Auswertung erfolgt dabei für Herzsignale zweckmäßigerweise, wie es in der deutschen Patentanmeldung P 27 16 739.1 beschrieben ist. Werden dabei ein oder mehrere Mikroprozessoren verwendet, so können diese mittels einer geeigneten Programmierung für die notwendigen mathematischen Operationnen zür Ausgabe der die Uterustätigkeit betreffenden Signale mitbenutzt werden.

Günstige Weiterbildungen der Erfindung sind in den Unteransprüchen gekennzeichnet. Zwei bevorzugte Ausführungs former der erfindungsgemäßen Vorrichtung sind in der Zeichnung dargestellt und werden nachfolgend näher beschrieben. Es zeigen:

Fig. 1 eine Darstellung der Signalaufnahme,
Fig. 2 ein Blockschaltbild einjr ersten Ausführungsform der erfindungsgemäßen Vorrichtung, bei der gleichzeitig Mittel vorgesehen sind, um die fetale bzw. maternelle Herztätigkeit zu ermitteln,

Fig. 3 ein Schaltbild eines Verstärkers für das elektromyographisch aufgenommene abdominale Signal als Darstellung eines Details der in Fig. 2 schematisch dargestellten Vorrichtung,

Fig. 4 ein Schaltbild des Verarbeitungsteils für das Elektromyogramm, ebenfalls als Detaildarstellung eines Blocks der schematischen Darstellung gemäß Fig. 2 sowie

Fig. 5 ein Blockschaltbild einer zweiten Ausführungsform der Erfindung gemäß Fig. 2, mit Mitteln zur Befreiung der zu verarbeitenden Signale von stören den maternellen Herzsignalen (QRS-Komplexen).

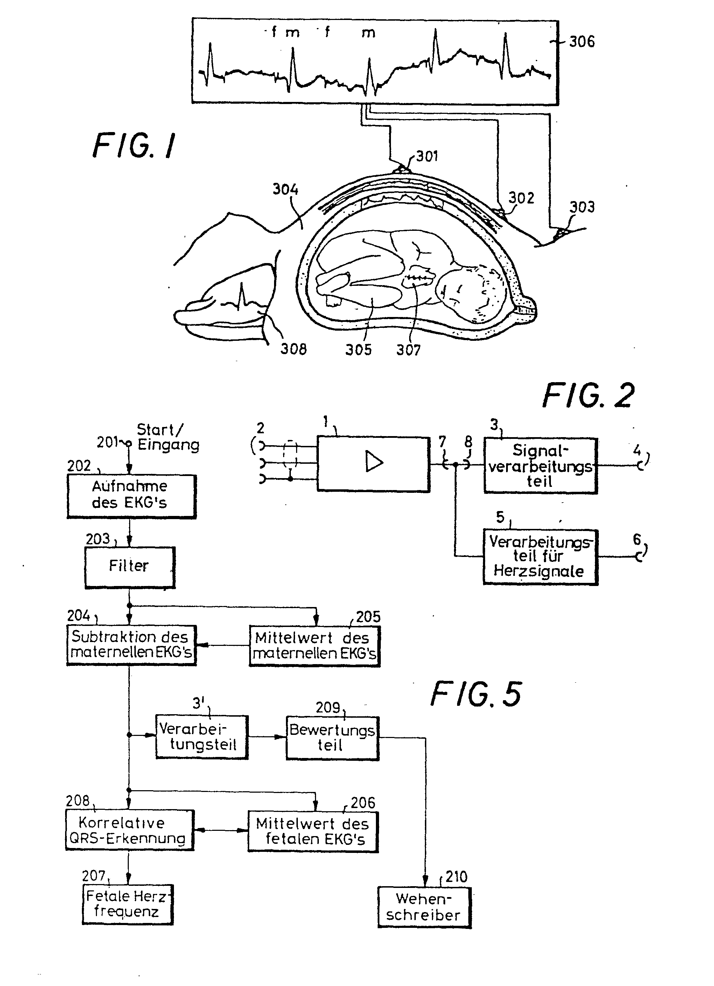

In Fig. 1 ist eine Darstellung der Signalaufnahme wiedergegeben. Verschiedene Elektroden 301, 302 und 303 sind zur Meßwertaufnahme am Körper einer Patientin 304 angebracht, deren Wehentätigkeit erfaßt werden soll. Bei einer Weiterbildung der Erfindung wird dabei gleichzeitig die Herztätigkeit eines Feten 305 untersucht. In dem mittels der Haut-Elektroden 301 bis 303 elektromyographisch aufgenommenen Signalverlauf 306 ist das Signal vom Herzen 307 des Feten vom Signal des Herzens 308 der Mutter sowie verschiedenen Störanteilen überlagert. Das fetale bzw. das maternelle Herzsignal ist im Signalverlauf 306 mehrfach mit "f" bzw. "m" gekennzeichnet. Die die Wehentätigkeit repräsentierenden Signale sind im aufgenommenen Signal nicht direkt erkennbar und werden erst durch die erfindungsgemäße Vorrichtung als Kurvenverlauf darstellbar.

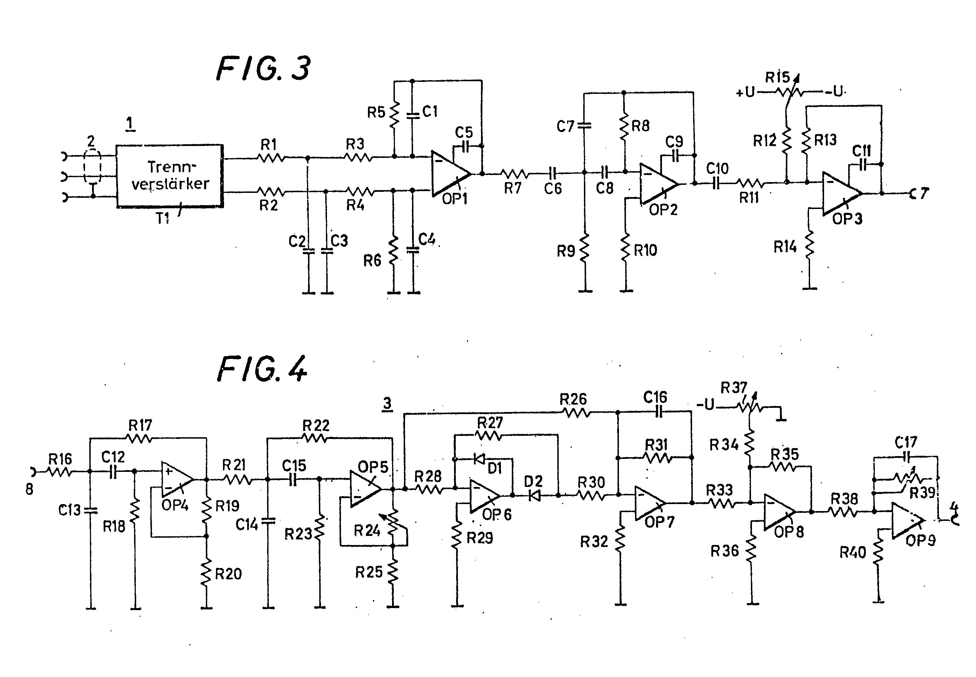

Die in Fig. 2 dargestellte erste Ausführungsform der erfindungsgemäßen Vorrichtung ermöglicht die Ermittlung und Aufzeichnung der Uterusaktivität durch die Erkennung und Auswertung von Muskelkontraktionen im Abdominalbereich. Der Eingang eines Verstärkers 1, der im einzelnen in Fig. 3 schaltungsmäßig dargestellt ist, ist über seine Eingänge 2 mit den hier nicht dargestellten Elektroden verbunden.

Das Signal vom Ausgang 7 des Verstärkers 1 wird einem Eingang 8 eines Verarbeitungsteils 3 zugeleitet, mittels dessen die die Uterustätigkeit betreffenden Signalanteile so aufbereitet werden, daß das einem Ausgang 4 zugeleitete Signal des Verarbeitungsteils 3 ein Maß für die Uterusmotilität darstellt. Mit dem Ausgang 4 ist ein nicht dargestelltes Registriergerät verbunden, welches den Verlauf der Intensität der Wehentätigkeit in Abhängigkeit von der Zeit aufzeichnet, so daß der Arzt aus den sich ergebenden charakteristischen Kurvenverläufen, gegebenenfalls durch Vergleich mit in einem Verzeichnis aufgeführten Kurvenverläufen, Rückschlüsse auf die Wehentätigkeit ziehen kann. Die Schaltung des Verarbeitungsteils 3 ist im einzelnen in Fig. 4 dargestellt.

Bei der dargestellten Ausführungsform der erfindungsgemäßen Vorrichtung ist die Möglichkeit vorgesehen aus dem mittels einer einzigen Elektroden-Anordnung aufgenommenen Signalverlauf gleichzeitig Aufschluß über die Herztätigkeit zu gewinnen. Das Signal am Ausgang 7 des Verstärkers 1 wird dazu zu einem Verarbeitungsteil 5 für Herzsignale abgezweigt. (Die Möglichkeiten zur Trennung der beiden Signale werden dabei weiter unten näher erläutert.) Am Ausgang 6 des Verarbeitungsteils 5 stehen das fetale Elektrokardiogramm oder ein Signal als Maß für die Herzfrequenz zur Verfügung und können beispeilsweise in ihrem zeitabhängigen Kurvenverlauf wiedergegeben werden. Die Darstellung der fetalen Herzsignale läßt sich mit derjenigen der Uterustätigkeit in einem Kardiotokogramm zusammenfassen, welches von einem einzigen graphischen Ausgabegerät dargestellt werden kann.

In Fig. 3 ist das vollständige Schaltbild des Verstärkers für das abdominale Signal (1 in Fig. 2) wiedergegeben. Über den Eingang 2 gelangen die Signale von den Abdominalelektroden zu einem Trennverstärker T 1, welcher zur Potentialtrennung von Patientin und Meßgerät dient. Die Ausgänge des Trennverstärkers T 1 sind mit den Eingängen eines Differenzverstärkers verbunden, welcher aus einem Operationsverstärker OP 1 besteht, der mit einer Anzahl von Widerständen und Kondensatoren beschaltet ist. Die Widerstände R 1 und R 3 bzw. R2 und R 4 bilden mit den Kondensatoren C 2 bzw. C 3 jeweils einen EingangsTiefpaß, während die Widerstands-Kondensator-Kombinationen C 1/R 5 und C 4/R 6 sowie der Kondensator C 5 für einen Verstärkungsabfall zu hohen Frequenzen hin sorgen. Die nachfolgende Stufe, welche den Operationsverstärker OP 2 enthält, ist in üblicher Weise als selektives Filter geschaltet, wobei im dargestellten Ausführungsbeispiel die Werte der Widerstände R 7 bis R 10 und die der Kondensatoren C 6 bis C 9 so gewählt sind, daß die Schaltung vorzugsweise die tiefen Frequenzen des abdominalen Signals beschneidet. Die letzte einen Operationsverstärker OP 3 enthaltende Stufe arbeitet als reine wechselspannungsgekoppelte Verstärkerschaltung, wobei der Verstärkungsfaktor durch das Verhältnis der Widerstände R 11 und R 13 bestimmt wird und ein Nullabgleich des Ausgangssignals über den Widerstand R 12 mittels eines veränderlichen Widerstands R 15 erfolgen kann. Die Gesamtschaltung des Verstärkers für das abdominale Signal bewirkt eine erste Beschneidung der bei der nachfolgenden Signalverarbeitung störenden Frequenzen.

In Fig. 4 ist die Schaltung des Verarbeitungsteils für das Elektromyogramm (3 in Fig. 2) dargestellt. Durch die beiden ersten Stufen mit den Operationsverstärkern OP 4 und OP 5 wird eine Bandpaßfilterung des am Eingang des Verarbeitungsteils 3 erscheinenden Signals vorgenommen. Das Bandpaßfilter weist die Frequenzgrenzen $f_u = 150$ Hz und $f_o = 250$ Hz auf, da in diesem Frequenzbereich die leistung der uterinen Aktionspotentiale diejenige der Störanteile übertrifft. Die Beschaltung der Operationsverstärker OP 4 und OP 5 mit Widerständen R 16 bis R 26 und Kondensatoren C 12 bis C 15 entspricht in der Funktion derjenigen des Operationsverstärkers OP 2 in Fig. 3. Die Bemessung der werte der Bauelemente erfolgt nach den für derartige aktive Filterschaltungen gültigen Regeln. Eine Einstellung des Ausgangspegels und der Güte des Filters läßt sich mittels des veränderlichen Widerstands R 24 vornehmen.

Die Operationsverstärker OP 6 und OP 7 sind als integrierender Vollweg-Gleichrichter geschaltet. Das Aus-gangssignal stellt eine gute Näherung für den Effektiv-(RMS-)Wert der Aktionspotentiale dar, wobei die verwendete Schaltung im Gegensatz zu einer analogen Quadrierschaltung einfach aufgebaut ist und betriebssicher arbeitet. Die erste, den Operationsverstärker OP 6 enthaltende Stufe bildet einen Halbwellengleichrichter, während die zweite Stufe mit dem Operationsverstärker OP 7 als integrierender Addierer zu dem über den Widerstand R 26 an seinem Eingang erscheinenden Eingangssignal der vorangehenden Stufe über den Widerstand R 30 die gleichgerichteten Halbwellen aus der vorangehenden Stufe mit negativem Vorzeichen addiert. Bei dieser einfach gehaltenen Ausführungsform bilden die gleichgerichteten Signale ein Maß für die Intensität der Wechselspannungsanteile. Bei anderen Ausführungen wird die Gleichrichterschaltung durch eine Schaltung zur Ermittlung der Leistung der Signale bzw. zur Bildung des Effektivwerts ersetzt. Derartige Schaltungen stehen in analoger bzw. digitaler Ausführung als fertige Komponenten zur Verfügung. Der Kondensator C 16 und der Widerstand R 31 geben der Schaltung den zur Mittelwerbildung erforderlichen Tiefpaßcharakter.

Der Wert der Zeitkonstanten beträgt einige Sekunden und bestimmt einerseits die Wiedergabe des zeitlichen Verlaufs der Uterusaktivitäten (eine kleine Zeitkonstante ergibt dabei eine große zeitliche Auflösung, während eine größer gewählte Zeitkonstante einzelne Wehen deutlicher hervortreten läßt) und andererseits den dabei auswertbaren Frequenzbereich. Mit einer größer gewählten Zeitkonstanten (größer als ungefähr 10 Sekunden) ergibt sich die vorteilhafte Möglichkeit, auch oder ausschließlich diejenigen Frequenzbereich für die Ermittlung der Uterustätigkeit auszunutzen, welche von den Herzsignalen überlagert sind. Damit ist eine konstruktiv besonders einfache Ausführungsform möglich, bei der auf getrennte Filtermittel zum Beseitigen des bei der Uterusaktivität störenden maternellen Herzsignals verzichtet werden kann. Bei einer anderen Ausführungsform der Erfindung ist an dieser Stelle eine auf die Gleichrichterschaltung folgende Stufe zur digitalen bzw. analogen Bildung des quadratischen Mittelwerts der Signale eingefügt.

Mit der nachfolgenden, den Operationsverstärker OP 8 enthaltenden Stufe läßt sich mittels des veränderlichen Widerstandes R 37 die Nullinie des Ausgangssignals einstellen. Im übrigen dient diese Stufe als Verstärker mit einem Verstärkungsfaktor, der dem Verhältnis der Werte der Widerstände R 35 und R 33 entspricht. Die letzte, den Operationsverstärker OP 9 enthaltenden Stufe bildet einen Endverstärker für das Ausgangssignals, wobei der Pegel über den Widerstand R 39 einstellbar ist. Der Kondensator C 17 bewirkt eine zusätzliche Absenkung des oberen Frequenzbereiches, so daß eine an den Ausgang des Operationsverstärkers OP 9 angeschlossene Schreibvorrichtung vor stoßartigen Ausschlägen geschützt ist.

Bei der im Vorangehenden dargestellten Ausführungsform der erfindungsgemäßen Vorrichtung wird in vorteilhafter Weise von der Tatsache Gebrauch gemacht, daß ein begrenztes Frequenzband wesentliche für die Uterusmotilität charakteristische Signale enthält. Dadurch ist es einerseits möglich, eine einfach und sicher arbeitende Vorrichtung zur Detektion dieser Signale zu schaffen, welche die Nutzsignale unter Benutzung unkomplizierter Mittel, wie Frequenzfilter, von den Störsignalen trennt. Dabei kann vorher gegebenenfalls auch eine Abtrennung der separat auszuwertenden fetalen und maternellen Herzsignale vorgenommen werden, wie es schematisch in Fig. 2 dargestellt ist, wobei der für die Herztätigkeit charakteristische Frequenzbereich ungefähr zwischen 15 und 40 Hz liegt. Das gesamte von den Elektroden aufgenommene Signal S(t) setzt sich zusammen gemäß .

$$S(t) = D(t)[E_f(t) + E_m(t) + EMG + N(t)],$$

wobei
D(t) einen multiplikativen Verzerrungsfaktor,
$E_f(t)$ das fetale Herzsignal,
$E_m(t)$ das maternelle Herzsignal,
EMG den für die Uterustätigkeit charakteristischen Anteil des Elektromyogramms und
N(t) einen additiven Störanteil darstellt.

Der Einfluß von D(t) ist sehr gering und kann ohne Nachteil für das Meßergebnis vernachlässigt werden.

Wird die Zeitkonstante für die zeitliche Mittelung ausreichend groß gewählt, so kann auf eine frequenzmäßige Trennung von für die Uterus- und die Herztätigkeit charakteristischen Signalanteilen verzichtet werden, da bei Werten der Zeitkonstanten von über ungefähr 10 Sekunden die Herzsignale bei der Auswertung der Uterusaktivität—wie oben erwähnt—nicht mehr störend in Erscheinung treten. Das im oberen Teil der Fig. 4 dargestellte Bandpaßfilter kann damit entfallen, während ein entsprechendes Filter für den Frequenzbereich von ungefähr 15 bis 40 Hz, das ursprünglich im Verarbeitungsteil 5 vorgesehen ist, dem Verstärker 1 zugeordnet wird, so daß damit insgesamt nur ein Bandfilter erforderlich ist.

Unter Ausfilterung des EMG-Anteils kann das am Eingang des Signalverarbeitungsteiles anliegende Signal vorzugsweise so verarbeitet werden, wie es in der DE—A 27 16 739 (veröffentlicht: 26. 10. 1978) und in Fig. 5 schematisch dargestellt ist, die eine grundsätzliche Ausführungsform der Erfindung beschreibt, bei der die Beseitigung störender sich wiederholender Signalanteile und die Detektion der fetalen Herzfrequenz in der Weise vorgenommen wird, wie es in der genannten Patentanmeldung ausgeführt ist.

Punkt 201 kann dabei entweder den Eingang einer elektrischen Schaltung oder entsprechend den Startpunkt eines Rechenprogramms bilden. Die nachfolgenden Blöcke können entweder elektrische Schaltungsstufen oder geeignete Programmblöcke bilden. Im Folgenden soll die schaltungsmässige Interpretation zugrundegelegt werden. Stufe 202 umfaßt dabei alle Schritte, die mit der Aufnahme des elektromyographischen Signals zusammenhängen.

Das myographisch aufgenommene Signal wird in einer Filterstufe 203 von störenden Signalanteilen befreit, wobei in der nachfolgenden Stufe 204 das maternelle Herzsignal durch Subtraktion aus dem gefilterten Eingangssignal beseitigt wird. Subtrahiert wird ein Signalverlauf, der einem mittleren maternellen EKG entspricht, wobei das maternelle EKG erkannt und durch Mittelwertbildung in seinem Verlauf laufend verbessert bzw. aktualisiert wird. Das mittlere maternelle EKG wird dabei in Stufe 205 festgehalten. Bei der Subtraktion wird die Amplitude des zu subtrahierenden Signals an die Amplitude des Eingangssignals anpaßt.

Das derart von störenden Anteilen befreite Signal dient als Eingangssignal für einen Signal-Verarbeitungsteil 3', in dem die Verarbeitung der die Uterustätigkeit betreffenden Signale, wie anhand des Verarbeitungsteils 3 beschrieben, vorgenommen wird. (Im Gegensatz zu dem in Fig. 4 dargestellten Verarbeitungsteil 3 ist bei dem Verarbeitungsteil 3' aber die Bandfilterbaugruppe weggelassen, da die Filterung gemeinsam durch das Filter 203 erfolgt.) In einem Bewertungsteil 209 kann eine logarithmische oder auch potenzierende Verzerrung der ausgegebenen Amplitudenwerte erfolgen, die mittels eines Wehenschreibers 210 zeitabhängig graphisch dargestellt werden.

In einer in dem anderen Signalweg nachfolgenden Stufe 206 wird die eigentliche Ermittlung des fetalen EKG-Signals vorgenommen, wobei zunächst das Auftreten eines fetalen Herzsignals durch ein Maximumkriterium festgestellt wird, während dann ein zweites Kriterium zur Ermittlung des Auftretens eines fetalen Herzsignals im Maß der Übereinstimmung zwischen dem Verlauf des Eingangssignals und dem in seinem Amplitudenverlauf gespeicherten Mustersignal besteht, das seinerseits durch Mittelwertbildung fortlaufend an die aktuelle Signalform angepaßt wird. Der Mittelwert des fetalen EKG (QRS-Komplex) ist in Stufe 206 festgehalten, während in Stufe 208 aus dem zeitlichen Abstand der fetalen Herzsignale die fetale Herzfrequenz errechnet und festgehalten wird.

Die in den Fig. 3 und 4 dargestellte Ausführungsform der erfindungsgemäßen Vorrichtung ist mit analogen Bauelementen realisiert. Diese Ausführung ist dann besonders günstig, wenn sie für sich allein benutzt werden soll. In einer digitalen Ausführung, bei der bei Verwendung im Zusammenhang mit anderen Erfassungseinrichtungen für Patientendaten vorhandene Datenverarbeitungseinrichtungen, wie Mikroprozessoren, vorteilhaft mitbenutzbar sind, können für die erforderlichen mathematischen Berechnungen (Mittelwertbildung, Leistungs-, RMS-Ermittlung, Logarithmierung, Potenzbildung) Programme oder Programmtiele benutzt werden, wie sie beispielsweise in den für die entsprechenden Datenverarbeitungseinrichtungen oder Mikroprozessoren (z.B. LSI 11 oder 8080) zur Verfügung stehenden Programmsammlungen enthalten sind.

**Patentansprüche**

1. Schaltungsanordnung zur Detektion und Registrierung der Uterusaktivität mittels elektromyographisch im abdomialen Bereich extern abgeleiteter Signale mit Filtermitteln zum Ausfiltern von Wechselspannungsanteilen (OP 1, 2, 4, 5) für die weitere Signalverarbeitung dadurch gekennzeichnet, daß die ausgefilterten Wechselspannungsanteile im Frequenzbereich von ungefähr 10 bis 300 Hz liegen, und daß eine Einrichtung (3, 3') zur Erzeugung von Signalen als Maß für die Intensität der ausgefilterten Wechselspannungsanteile vorgesehen ist, die eine Schaltung (OP 6 bis 9 mit C 16, R 31) zur zeitlichen Mitteilung der vorgenannten Spannunganteile zum Erhalt eines die Größe des intrauterinen Druck repräsentierenden Signals umfaßt.

2. Schaltüngsanordnüng nach Anspruch 1, dadurch gekennzeichnet, daß die Einrichtüng zur Erzeugung von Signalen Signale abgibt, die im wesentlichen ein Maß für die elektrische Leistung des Wechselspannungsanteils des elektromyographisch abgeleiteten Signals darstellen.

3. Schaltüngsanordnüng nach Anspruch 1, dadurch gekennzeichnet, daß Mittel zur Gleichrichtung der Wechselspannungsanteile (OP 6, OP 7) vorgesehen sind.

4. Schaltüngsanordnüng nach Anspruch 3, dadurch gekennzeichnet, daß die Mittelung im wesentlichen quadratisch erfolgt.

5. Schaltüngsanordnüng nach Anspruch 1, dadurch gekennzeichnet, daß die Einrichtung zur Erzeugung von Signalen Signale abgibt, die im wesentlichen dem Effektiv-(RMS-)Wert des Wechselspannungsanteils der elektromyographisch abgeleiteten Signale entsprechen.

6. Schaltüngsanordnüng nach einem der vorangehenden Ansprüche, dadurch gekennzeichnet, daß die Einrichtung zur zeitlichen Mittelung einen Tiefpaß (OP 7 und 9) aufweist.

7. Schaltüngsanordnüng nach Anspruch 6, dadurch gekennzeichnet, daß die Zeitkonstante des Tiefpasses mehrere Sekunden beträgt.

8. Schaltungsanordnüng nach einem der vorangehenden Ansprüche, dadurch gekennzeichnet, daß der ausgefilterte Frequenzbereich im wesentlichen innerhalb der Grenzen von 150 und 250 Hz liegt.

9. Schaltungsanordnüng nach einem der vorangehenden Ansprüche, dadurch gekennzeichnet, daß Mittel zur Ausgabe gemittelten Signals in logarithmischer Darstellung vorgesehen sind.

10. Schaltüngsanordnüng nach einem der Ansprüche 1 bis 8, dadurch gekennzeichnet, daß Mittel zur Ausgabe des gemittelten Signals in potenzierter Darstellung vorgesehen sind.

11. Schaltüngsanordnüng nach einem der vorangehenden Ansprüche, dadurch gekennzeichnet, daß für die elektromyographische Erfassung von Meßsignalen zur Detektion und Registrierung von Uterusaktivität und Herzsignalen gemeinsame Meßwertaufnehmer (Elektroden 301 bis 303) und/oder gemeinsame Auswertungsmittel vorgesehen sind.

12. Schaltungsanordnung nach Anspruch 11, dadurch gekennzeichnet, daß die über gemeinsame Meßwertaufnehmer myographisch erfaßten Signale für die Auswertung durch Bandfiltermittel im wesentlichen auf einen Frequenzbereich von 15 bis 40 Hz begrenzt werden.

13. Schaltungsanordnung nach einem der Ansprüche 11 oder 12, dadurch gekennzeichnet, daß Mittel vorgesehen sind, welche die Signalauswertung beeinträchtigende wiederkehrende Störsignale erkennen, in ihrem Amplitudenverlauf in einem Speicher festhalten und, wenn das Auftreten eines Störsignals im Eingangssignal entdeckt ist, von diesem amplitudenmäßig phasenrichtig subtrahieren.

14. Schaltungsanordnung nach Anspruch 13 zur Ermittlung fetaler Herzsignale, dadurch gekennzeichnet, daß es sich bei dem wiederkehrenden Störsignal um den maternellen QRS-Komplex handelt.

15. Schaltungsanordnung nach einem der Ansprüche 11 bis 14, dadurch gekennzeichnet, daß zur Entdeckung von QRS-Komplexen fetaler Herzsingale im von den Signalen zur Detektion und Registrierung der Uterusaktivität getrennten Eingangssignal ein Speicher zum Festhalten eins nach einem ersten Kriterium erkannten fetalen QRS-Komplexes in seinem Amplitudenverlauf vorgesehen ist und letzterer als Muster zur Identifikation nach folgender QRS-Komplexe nach einem zweiten Kriterium herangezogen wird, das in dem Maß der Übereinstimmung des jeweils anliegenden Signals mit dem Amplitudenverlauf des als Muster festgehaltenen fetalen QRS-Komplexes besteht.

16. Schaltüngsanordnüng nach Anspruch 15, dadurch gekennzeichnet, daß Mittel vorgesehen sind, um den als Muster festgehaltenen fetalen QRS-Komplex in seinem Amplitudenverlauf fortwährend an die aktuelle Form des fetalen QRS-Komplexes anzupassen.

## Claims

1. A circuit arrangement for detecting and registering activity in the uterus with the aid of externally derived electromyographic signals in the abdominal region and having filter means for filtering out ac voltage components (OP 1, 2, 4, 5) for further signal processing characterised in that the components of the ac voltage which are filtered out are in the frequency range of approximately 10 to 300 Hz, and that there is provided a device (3, 3') for producing signals as a measure of the intensity of the filtered out ac voltage components, the device including a circuit (OP 6 to 9 with C 16, R31) for time-averaging the said voltage components in order to obtain a signal which represents the magnitude of the intra-uterine pressure.

2. A circuit arrangement according to claim 1, characterised in that the device for producing signals emits signals which essentially represent a measure of the electrical power of the ac voltage component of the electromyographically derived signal.

3. A circuit arrangement according to claim 1 characterised in that means are provided to rectify the ac voltage components (OP 6, OP 7).

4. A circuit arrangement according to claim 3, characterised in that averaging takes place essentially quadratically.

5. A circuit arrangement according to claim 1, characterised in that the device for producing signals emits signals which correspond essentially to the effective (RMS) value of the ac voltage component of the electromyographically derived signals.

6. A circuit arrangement according to any one of the preceding claims characterised in that the device for time averaging has a low pass filter (OP 7 and 9).

7. A circuit arrangement according to claim 6, characterised in that the time constant of the low pass filter is several seconds.

8. A circuit arrangement according to any one of the preceding claims, characterised in that the filtered out frequency range is essentially within the limits of 150 and 250 Hz.

9. A circuit arrangement according to any one of the preceding claims, characterised in that means are provided for emitting the averaged signal in logarithmic display.

10. A circuit arrangement according to any one of claims 1 to 8, characterised in that means are provided for output of the averaged signal shown in a form in which it is raised to a higher power.

11. A circuit arrangement according to any one of the preceding claims, characterised in that measured value pick ups (electrodes 301 to 303) and/or evaluating means are provided for jointly detecting measured signals for detecting and registering uterus activity and heart signals.

12. A circuit arrangement according to claim 11, characterised in that the signals detected myographically by means of the common measured value pick ups for evaluation are essentially limited to a frequency range of 15 to 40 Hz by band filter means.

13. A circuit arrangement according to claim 11 or 12, characterised in that means are provided which detect recurring interference signals which restrict the evaluation of the signal, hold their amplitude curve in a store, and, whenever an interference signal is discovered in the input signal, subtract it from the input signal in amplitude and in correct phase.

14. A circuit arrangement according to claim 13 for determining foetal heart signals, characterised in that the recurring interference signal is the maternal QRS complex.

15. A circuit arrangement according to any one of claims 11 to 14, characterised in that in order to detect QRS complexes of foetal heart signals in the input signal which is separate from the signals for detecting and registering the uterus activity, a store is provided for holding a foetal QRS complex in its amplitude curve, said complex being detected according to a first criterion, and said foetal complex is used as a standard for identifying the following QRS complexes according to a second criterion, and this second criterion constitutes the measure of coincidence of the respective signal with the amplitude curve of the foetal QRS complex which is stored as a standard.

16. A circuit arrangement according to claim 15, characterised in that means are provided in order to continuously match the foetal QRS complex stored as a standard in its amplitude curve to the current form of the foetal QRS complex.

**Revendications**

1. Montage pour la détection et l'enregistrement de l'activité utérine au moyen de signaux recueillis extérieurement dans la région abdominale par voie électromyographique, comprenant des moyens de filtrage pour éliminer des composantes de tension alternative (OP 1, 2, 4, 5) en vue du traitement consécutif du signal, caractérisé en ce que les composantes de tension alternative éliminées par filtrage sont comprises dans la gamme de fréquences d'environ 10 à 300 Hz et en ce qu'un dispositif (3, 3') est prévu pour produire des signaux représentant une mesure de l'intensité des composantes de tension alternative éliminées par filtrage, ce dispositif comprenant un montage (OP 6 à 9 et C 16, R 31), pour la prise de lay moyenne dans le temps desdites composantes de tension afin d'obtenir un signal représentatif de la grandeur de la pression intra-utérine.

2. Montage selon la revendication 1, caractérisé en ce que le dispositif pour produire des signaux délivre des signaux qui représentent essentiellement une mesure de la puissance électrique de la composante de tension alternative du signal recueilli par voie électromyographique.

3. Montage selon la revendication 1, caractérisé en ce que des moyens sont prévus pour redresser les composantes de tension alternative (OP 6, OP 7).

4. Montage selon la revendication 3, caractérisé en ce que la prise de la moyenne s'effectue essentiellement par élévation au carré.

5. Montage selon la revendication 1, caractérisé en ce que le dispositif pour produire des signaux délivre des signaux qui correspondent essentiellement à la valeur effective (valeur RMS) de la composante de tension alternative des signaux recueillis par voie électromyographique.

6. Montage selon l'une des revendications précédentes, caractérisé en ce que le dispositif pour la prise de la moyenne dans le temps comporte un filtre passe-bas (OP 7 et 9).

7. Montage selon la revendication 6, caractérisé en ce que la constante de temps du filtre passe-bas est de plusieurs secondes.

8. Montage selon l'une des revendications précédentes, caractérisé en ce que la gamme de fréquences obtenue par élimination par filtrage est essentiellement comprise dans les limites de 150 et 250 Hz.

9. Montage selon l'une des revendications précédentes, caractérisé en ce que des moyens sont prévus pour délivrer la moyenne du signal sous une forme logarithmique.

10. Montage selon l'une des revendications 1 à 8, caractérisé en ce que des moyens sont prévus pour délivrer la moyenne du signal sous une forme élevée à une puissance plus haute.

11. Montage selon l'une des revendications précédentes, caractérisé en ce que des capteurs de valeur de mesure communs (électrodes 301 à 303) et/ou des moyens d'exploitation communs sont prévus pour recueillir par voie électromyographique des signaux de mesure servant à la détection et à l'enregistrement de l'activité utérine et de signaux cardiaques.

12. Montage selon la revendication 11, caractérisé en ce que les signaux recueillis par voie myographique par des capteurs communs sont, en vue de l'exploitation, limités essentiellement à une gamme de fréquences de 15 à 40 Hz par des moyens de filtrage passe-bande.

13. Montage selon la revendication 11 ou 12, caractérisé en ce que des moyens sont prévus pour reconnaître des signaux parasites répétitifs affectant l'exploitation du signal, pour retenir la variation de leur amplitude dans une mémoire et pour, à la détection d'un signal parasite dans le signal d'entrée, soustraire le signal parasite quant à son amplitude et avec la phase correcte du signal d'entrée.

14. Montage selon la revendication 13 pour la détermination de signaux cardiaques foetaux, caractérisé en ce que le signal parasite répétitif est le complexe QRS maternel.

15. Montage selon l'une des revendications 11 à 14, caractérisé en ce que, pour la dètection de complexes QRS de signaux cardiaques foetaux dans le signal d'entrée séparé des signaux pour la détection et l'enrigistrement de l'activité utérine, une mémoire est prévue pour retenir la variation d'amplitude d'un complexe QRS foetal reconnu selon un premier critère et en ce que cette variation d'amplitude est utilisée comme modèle pour l'identification de complexes QRS suivants selon un second critère qui consiste dans le degré de concordance entre le signal appliqué chaque fois et la variation d'amplitude du complexe QRS foetal retenu comme modèle.

16. Montage selon la revendication 15, caractérisé en ce que des moyens sont prévus pour adapter continuellement la variation d'amplitude du complexe QRS foetal retenu comme modèle à la forme actuelle du complexe QRS foetal.

## FIG. 1

306

f m f m

301

304

302

303

305 307

308

## FIG. 2

2

1

7 8

3
Signalverarbeitungsteil

4

5
Verarbeitungsteil für
Herzsignale

6

## FIG. 5

Start/
201 Eingang

202
Aufnahme
des EKG's

203
Filter

204
Subtraktion des
maternellen EKG's

205
Mittelwert des
maternellen EKG's

3'
Verarbeitungsteil

209
Bewertungsteil

208
Korrelative
QRS-Erkennung

206
Mittelwert des
fetalen EKG's

207
Fetale Herzfrequenz

210
Wehenschreiber

1

FIG. 3

FIG. 4